# EUROPÄISCHE PATENTSCHRIFT

(11) **EP 0 371 303 B1**
(45) Veröffentlichungstag und Bekanntmachung des Hinweises auf die Patenterteilung: **27.04.1994**
(21) Anmeldenummer: 89120963.7
(22) Anmeldetag: 11.11.1989
(51) Int. Cl.: A61N 5/10, G21K 1/02

(54) **Strahlentherapiegerät**
Radiation therapy apparatus
Appareil d'actinothérapie

(30) Priorität: 29.11.1988 CH 4427/88
(43) Veröffentlichungstag der Anmeldung: 06.06.1990
(73) Patentinhaber: Varian International AG., CH-6300 Zug (CH)
(72) Erfinder: Vogt, Heinz, CH-5422 Oberehrendingen (CH); Klasen, René, CH-8427 Rorbas (CH); Schär, Hugo, CH-8416 Flaach (CH)
(74) Vertreter: Hetzer, Hans Jürgen

(56) Entgegenhaltungen:
- EP-A- 0 193 509
- EP-A- 0 196 138
- DE-A- 2 753 397
- DE-A- 3 621 868
- DE-B- 1 037 035
- FR-A- 2 524 690

## Beschreibung

Die Erfindung betrifft ein Strahlentherapiegerät mit einem von einem Fokuspunkt her entlang einer Strahlachse sich ausbreitenden Strahlenbündel und einem auf der Strahlachse angeordneten Strahlerkopf mit folgenden Merkmalen:
a) der Strahlerkopf umfasst einen doppelt fokussierten Konturenkollimator (Multi Leaf Collimator);
b) der Konturenkollimator weist eine Mehrzahl von nebeneinander angeordneten Blendplatten auf, welche jeweils zwei Seitenflächen, zwei Frontflächen und eine Innen- und eine Aussenfläche haben;
c) es sind Mittel zum Verschieben jeder einzelnen Blendplatte vorhanden.

### Stand der Technik

Strahlentherapiegeräte werden in der Medizin eingesetzt, um Tumore mit hochenergetischen Photonen oder Elektronen therapeutisch zu behandeln. Dabei ist es wichtig, dass das vom Gerät erzeugte Strahlenbündel genau definierte Eigenschaften bezüglich Feldbegrenzung hat. Besonders gut geeignet sind in dieser Hinsicht Strahlentherapiegeräte mit Konturenkollimatoren der eingangs genannten Art.

Ein solches Gerät ist z.B. aus der Patentschrift US-A-4,672,212 bekannt. Anstelle eines konventionellen Paares von Kollimatorblöcken wird dort ein Konturenkollimator eingesetzt. Um unerwünschte Halbschatten am Rand des Strahlungsfeldes zu vermeiden, ist der Konturenkollimator doppelt fokussiert. Zu diesem Zweck sind die einzelnen Blendplatten Segmente eines Kreisrings und haben einen Querschnitt von der Form eines gleichschenkligen Trapezes. Der aus den Blendplatten zusammengesetzte Konturenkollimator ist also in zwei zueinander senkrechten Richtungen kreisförmig gebogen und imitiert damit einen Teil einer Kugelschale (allerdings nur näherungsweise).

Jede Blendplatte ist an ihrem hinteren Ende mit einem nach oben gerichteten Stab versehen. An diesem Stab wird die Blendplatte mit einem Motor vor- und zurückgeschoben.

Das Problem dieses Konturenkollimators liegt darin, dass zwischen den einzelnen Blendplatten eine unerwünschte Leckstrahlung auftritt. Ein weiterer Nachteil liegt in der komplizierten Ansteuerung. Die Blendplatten bewegen sich nämlich auf gekrümmten, von Platte zu Platte sich ändernden Bahnen. Die vorgeschlagene Kopplungsart zwischen Motor und Blendplatte führt zu einem nicht-linearen Zusammenhang zwischen Plattenvorschub und Motorendrehzahl.

Einen ganz anderen Konturenkollimator beschreibt die veröffentlichte Europäische Patentanmeldung EP-A-0 259 989. Dieser wird in einem konventionellen Strahlentherapiegerät, zusätzlich zu den zwei Paaren von Kollimatorblöcken, zum Abschirmen von empfindlichen Organen des Patienten eingesetzt. Der Konturenkollimator besteht aus einer Mehrzahl seitlich aneinanderliegender Blendplatten. Die im wesentlichen rechteckigen Platten sind an ihrer Vorderseite leicht abgerundet und haben an den Seitenflächen geradlinige Führungsnuten. Sie laufen auf geraden Bahnen und werden mittels flexibler Kabel von zugeordneten Motoren angetrieben.

Zwar besitzt dieser Konturenkollimator eine lineare Ansteuerung, ist aber aufgrund der fehlenden doppelten Fokussierung nur in Verbindung mit den herkömmlichen Kollimatorblöcken verwendbar.

### Darstellung der Erfindung

Aufgabe der Erfindung ist es, ein Strahlentherapiegerät der eingangs genannten Art zu schaffen, welches eine grosse Gestaltungsfreiheit des Strahlungsfeldes zulässt. Gleichzeitig soll die Ansteuerung der Blendplatten möglichst einfach sein, und der Strahlerkopf soll schliesslich eine möglichst geringe Bauhöhe haben.

Erfindungsgemäss besteht die Lösung darin, dass bei einem Strahlentherapiegerät der eingangs genannten Art
- jede Seitenfläche jeder Blendplatte einen Teil einer Mantelfläche eines Kegels bildet, wobei alle solchen Kegel sowohl eine gemeinsame Kegelachse, welche senkrecht zur Strahlachse durch den Fokuspunkt verläuft, als auch eine gemeinsame Kegelspitze, welche mit dem Fokuspunkt zusammenfällt, haben, und
- dass Mittel zum Führen der Blendplatten vorgesehen sind, sodass jede Blendplatte beim Verschieben eine reine Rotation um die Kegelachse ausführt.

Der Kern der Erfindung liegt darin, dass die Blendplatten so geformt sind, dass sie mit ihren Seitenflächen formschlüssig aneinander liegen und dass gleichzeitig die doppelte Fokussierung beim Verschieben der Blendplatten erhalten bleibt. Im Gegensatz zum bekannten doppelt fokussierten Konturenkollimator hat jede Blendplatte eine individuelle Form, die gegeben ist durch ihre relative Position im ganzen Paket.

Bei einer besonders vorteilhaften Ausführungsform haben die Aussenflächen aller seitlich nebeneinander angeordneten Blendplatten insgesamt als Hüllfläche einen Teil einer Mantelfläche eines äusseren Zylinders, dessen Achse die Kegelachse ist. Die Mittel zum Verschieben der Blendplatten greifen an den Aussenflächen an. Auf diese Weise gestaltet sich die Ansteuerung besonders einfach.

Vorzugsweise umfassen die Mittel zum Verschieben für jede Blendplatte eine an der Aussenfläche der Blendplatte angebrachte Zahnschiene, ein darin eingreifendes Schnecken-Zahnstangen-Getriebe und einen dieses betätigenden Schrittmotor. Auf diese Weise ist eine lineare Beziehung zwischen der Drehzahl des Schrittmotors und dem Plattenvorschub gewährleistet.

Besonders vorteilhaft ist es, wenn der Strahlerkopf zwei übereinander angeordnete und senkrecht zueinander ausgerichtet Konturenkollimatoren aufweist und wenn auf der Strahlachse gegenüber dem Strahlerkopf eine Matrix-Ionisationskammer angeordnet ist, mit welcher die Konturenkollimatoren überwacht werden.

Weitere vorteilhafte Ausführungsformen ergeben sich aus den abhängigen Patentansprüchen.

### Kurze Beschreibung der Zeichnung

Nachfolgend soll die Erfindung anhand von Ausführungsbeispielen und im Zusammenhang mit der Zeichnung näher erläutert werden. Es zeigen:
- Fig. 1: ein Strahlentherapiegerät;
- Fig. 2: einen Strahlerkopf mit zwei erfindungsgemässen Konturenkollimatoren;
- Fig. 3a,b: eine Blendplatte von vorn resp. von der Seite gesehen;
- Fig. 4: eine Kollimatorhälfte mit 20 Blendplatten, deren Seitenflächen stufenförmig ausgebildet sind;
- Fig. 5: eine räumliche Darstellung der Bewegungsbahnen der Blendplatten;
- Fig. 6: eine dreidimensionale Darstellung einer Blendplatte;
- Fig. 7: eine Darstellung eines Strahlungsfeldes, wie es mit einem Strahlerkopf mit zwei senkrecht zueinander ausgerichteten Konturenkollimatoren erzeugt werden kann; und
- Fig. 8: eine Darstellung eines Strahlungsfeldes, wie es mit Konturenkollimatoren mit reduzierter Höhe erzeugt werden kann.

### Wege zur Ausführung der Erfindung

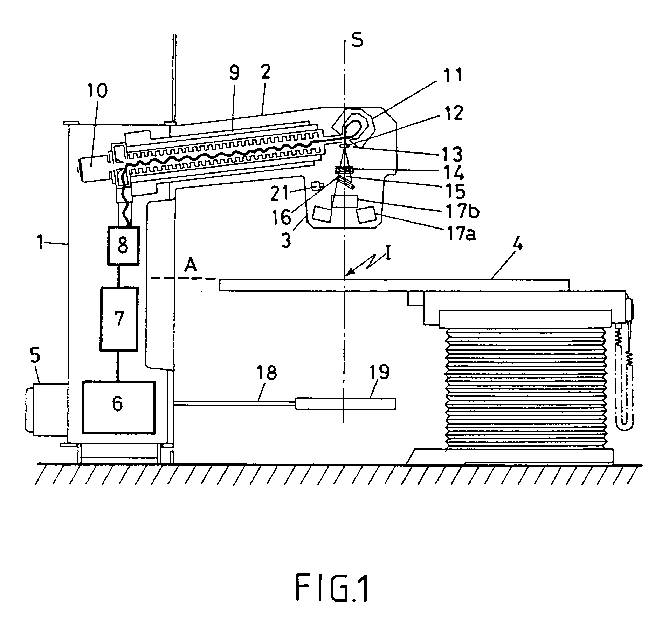

Fig. 1 zeigt ein Strahlentherapiegerät gemäss einer bevorzugten Ausführungsform der Erfindung. Es setzt sich zusammen aus einem Drehgestell 1, einem daran angebrachten Strahlerarm 2 und einem Strahlerkopf 3. Unter dem Strahlerkopf 3, liegt der zu behandelnde Patient auf einem Behandlungstisch 4.

Der Behandlungstisch 4 ist nicht Teil der Anmeldung. Er ist, wie es beim Stand der Technik üblich ist, in der Höhe verstellbar, um eine vertikale Achse drehbar und kann in Längs- und Querrichtung vor- und zurückgeschoben werden.

Das Drehgestell 1 ist um eine Achse A drehbar gelagert und wird von einem Antrieb 5 bewegt. Im Drehgestell 1 und im Strahlerarm 2 sind die zum Erzeugen von hochenergetischen Elektronen nötigen Bauteile untergebracht.

Eine von einem Impulstransformator 6 gesteuerte HF-Quelle 7 erzeugt Mikrowellen der benötigten Leistung. Ueber eine HF-Zuführung 8 werden die Mikrowellen einer Beschleunigungsröhre 9 zugeführt, welche im Strahlerarm 2 untergebracht ist. Eine Elektronenspritze 10 injiziert Elektronen in die Beschleunigungsröhre 9. Die Elektronen werden durch die Mikrowellen auf z.B. 8 MeV beschleunigt, in einem nachfolgenden Umlenkmagnet 11 energiemässig gefiltert und in Richtung einer Strahlachse S umgelenkt.

Die bis anhin beschriebenen Teile des Strahlentherapiegeräts sind als solche bekannt. Sie können durch andere Mittel ersetzt werden, welche zum Erzeugen eines energiereichen Elektronenstrahls geeignet sind.

Die Elektronen, die aus dem Umlenkmagnet 11 austreten, können entweder selbst zur Behandlung eingesetzt oder mit einem Target 12 in Photonen umgewandelt werden. Die erfindungsgemässen Teile des Strahlerkopfs entfalten ihre Wirkung unabhängig davon, ob bei der Therapie Photonen oder Elektronen oder andere Teilchen verwendet werden. Im folgenden wird deshalb nur noch von einem Strahlenbündel gesprochen.

In Fig. 1 sind die wesentlichen Teile des Strahlerkopfes 3 nur angedeutet. Ein Target 12 (zum Erzeugen der Photonen oder ein Elektronenstreufilter zum Streuen der Elektronen) befindet sich ausgangsseitig des Umlenkmagnets 11 erzeugt das Strahlenbündel. Ein wichtiger Bezugspunkt für die geometrisch optischen Ueberlegungen ist ein im folgenden als Fokuspunkt Q bezeichneter Punkt im dreidimensionalen Raum, von welchem aus sich das Strahlenbündel geometrisch optisch ausbreitet.

Ein anderer wichtiger Bezugspunkt im Raum ist das Isozentrum I. Es ist der Ort, an welchem das Strahlenbündel die gewünschte, optimale Wirkung entfaltet. Ueblicherweise trifft hier das Strahlenbündel auf den Tumor. Geometrisch betrachtet ist das Isozentrum I der Schnittpunkt der Strahlachse S mit der Drehachse A.

Das Strahlenbündel, welches sich also entlang der Strahlachse S ausbreitet, wird von einem Ausgleichsfilter 13 transformiert (z.B. in ein über das maximale Strahlungsfeld hin möglichst homogenes Strahlenbündel) und in einer Ionisationskammer 14 gemessen. Bevor das Strahlenbündel den Strahlerkopf 3 verlässt, wird es von zwei senkrecht zueinander ausgerichteten Paaren von Kollimatoren 17a,17b seitlich begrenzt.

Bei Bedarf kann ein Keilfilter 15 unterhalb der Ionisationskammer 14 ins Strahlenbündel geschoben werden. Ein Spiegel 16 und eine Lichtquelle 21 erlauben es, die seitliche Ausdehnung des Strahlenbündels simulationsmässig auf dem Patienten sichtbar zu machen.

Gemäss einer bevorzugten Ausführungsform ist auf der Strahlachse S gegenüber dem Strahlerkopf 3 und unterhalb des Isozentrums eine Matrix-Ionisationskammer 19 angeordnet. Sie ist mittels einer fixen oder einziehbaren Halterung 18 am Drehgestell 1 befestigt.

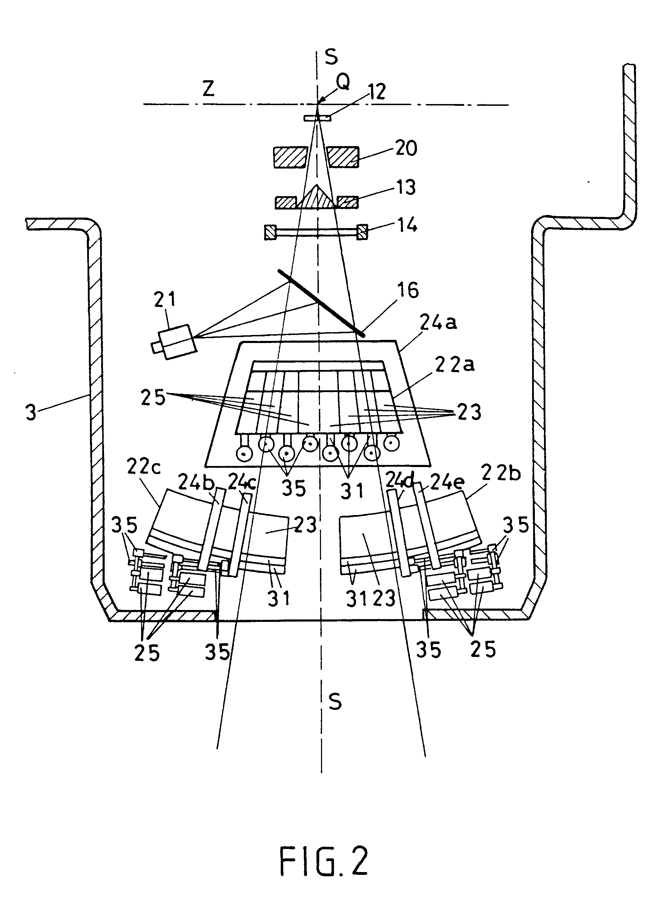

Fig. 2 zeigt den Strahlerkopf 3. Das Target 12 definiert auf der Strahlachse S den Fokuspunkt Q. Mit Z wird eine Kegelachse bezeichnet die senkrecht zur Strahlachse S durch den Fokuspunkt Q läuft.

Eine Strahlenblende 20 begrenzt das Strahlungsfeld auf eine Kreisfläche. Das Ausgleichsfilter 13 (engl. flattening filter) gleicht das Strahlungsfeld aus (z.B. Energie- resp Dosisspitzen im Zentrum). Anstatt eines einzigen können auch mehrere, auf einem Schieber befestigte Ausgleichsfilter vorgesehen sein. In gleicher Weise kann auch das Target 12 auswechselbar sein.

Nachfolgend werden die erfindungsgemässen Konturenkollimatoren erläutert. Für das Verständnis sind dabei die oben definierten geometrischen Bezugspunkte und -achsen von zentraler Bedeutung. Um die Beschreibung nicht unnötig zu komplizieren, werden Begriffe wie "oben" und "unten" resp. "über" und "unter(halb)" verwendet. Sie sind in dem Rahmen zu verstehen, dass der Fokuspunkt Q "oben" und das Isozentrum I "unten" ist. Das Strahlenbündel breitet sich also von oben nach unten aus. Entsprechend ist unter der Formulierung "Gegenstand X liegt über Gegenstand Y" zu verstehen, dass Gegenstand X näher beim Fokuspunkt ist als Gegenstand Y.

Fig. 2 zeigt eine Ausführungsform mit zwei übereinander liegenden und senkrecht zueinander ausgerichteten Konturenkollimatoren, welche jeweils zwei Kollimatorhälften 22a,22b,22c (die vierte Kollimatorhälfte ist zugunsten einer übersichtlichen Darstellung in Fig. 2 nicht eingezeichnet) umfassen. Jede Kollimatorhälfte 22a,22b,22c setzt sich aus einer Mehrzahl von nebeneinander angeordneten Blendplatten 23 zusammen, welche jeweils von zwei Haltebügeln 24a (der zweite Haltebügel der Kollimatorhälfte des oberen Konturenkollimators ist in Fig.2 nicht sichtbar), 24b und 24c, 24d und 24e individuell gestützt und geführt werden. Die Blendplatten 23 sind unabhängig voneinander verschiebbar. In Fig. 2 beispielsweise bewegen sich die Blendplatten des oberen Konturenkollimators senkrecht zur Zeichenebene und diejenigen des unteren Konturenkollimators parallel zur Zeichenebene.

Für jede Blendplatte 23 ist ein Schrittmotor 25 vorgesehen, welcher diese mittels eines Schnecken-Zahnstangen-Getriebes 35 und einer Zahnschiene 31 vor- und zurückschiebt. Die Schrittmotoren 25 eines Konturenkollimators, resp. der entsprechenden Kollimatorhälften, sind gestaffelt angeordnet, und zwar sowohl vertikal als auch horizontal.

Anstelle des Schrittmotors kann natürlich irgend ein anderer Antrieb (z.B. ein Gleichstrommotor mit Winkelgeber) verwendet werden.

Die Haltebügel führen einerseits die Blendplatten und stützen andererseits die Schnecken-Zahnstangen-Getriebe. Dabei wird jede Blendplatte von den zwei Haltebügeln einer Kollimatorhälfte an vier Punkten gestützt und geführt.

Der Vorteil des Schrittmotors liegt darin, dass er ständig unter Spannung und damit unter Kontrolle steht und dass er stets in einer definierten Position stehen bleibt. Folglich ist es möglich allein schon über die Schrittmotoren (ohne Potentiometer) die Stellung der Blendplatten zu erfassen.

Das Schnecken-Zahnstangen-Getriebe ist selbsthemmend. Damit entfällt eine zusätzliche Bremsvorrichtung, welche garantiert, dass die Blendplatten sich nicht selbständig verschieben können.

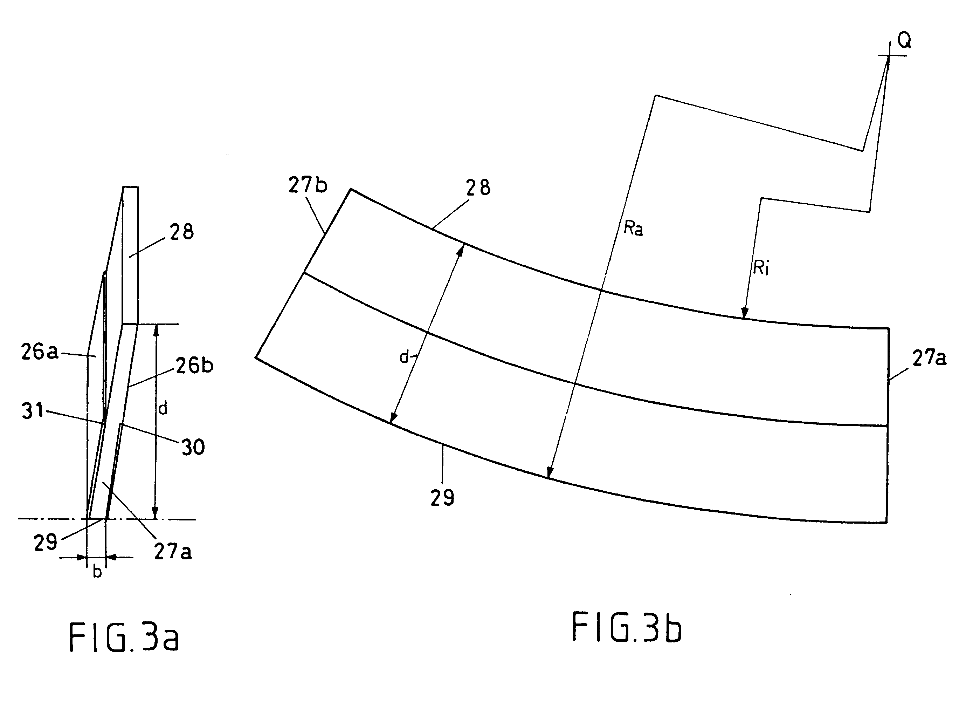

Fig.3a und 3b zeigen eine einzelne Blendplatte von vorn resp. von der Seite. Diese hat zwei Seitenflächen 26a, 26b, zwei Frontflächen 27a, 27b, eine Innenfläche 28 und eine Aussenfläche 29. Die Projektion der Seitenflächen 26a,26b auf eine zur Kegelachse (siehe später) senkrechte Ebene hat die Form eines Kreisringsegments. Im Querschnitt hat die Blendplatte die Form eines ungleichschenkligen Trapezes, wobei die parallelen Seiten des Trapezes durch die Innen- und die Aussenfläche 28, 29 und die ungleichen Schenkel des Trapezes durch die Seitenflächen 26a, 26b gegeben sind. Die Blendplatte hat eine Höhe d und eine Dicke b.

Die Innenfläche 28 ist etwas schmaler als die Aussenfläche 29, und zwar genau um soviel, dass eine Verlängerung einer Kante zwischen Seitenfläche 26a und Frontfläche 27a und eine Verlängerung einer Kante zwischen Frontfläche 27a und Seitenfläche 26b (d.h. eine Verlängerung der Schenkel des Trapezes) sich im Fokuspunkt Q (siehe Fig. 2) schneiden.

Um eine allfällige Leckstrahlung zwischen den Blendplatten zu minimieren, können die Seitenflächen 26a, 26b mit einander korrespondierenden Stufen 30, 31 versehen sein. Diese würden parallel zur Innen- resp. Aussenfläche 28 resp. 29 verlaufen, d.h. sie wären kreisbogenförmig.

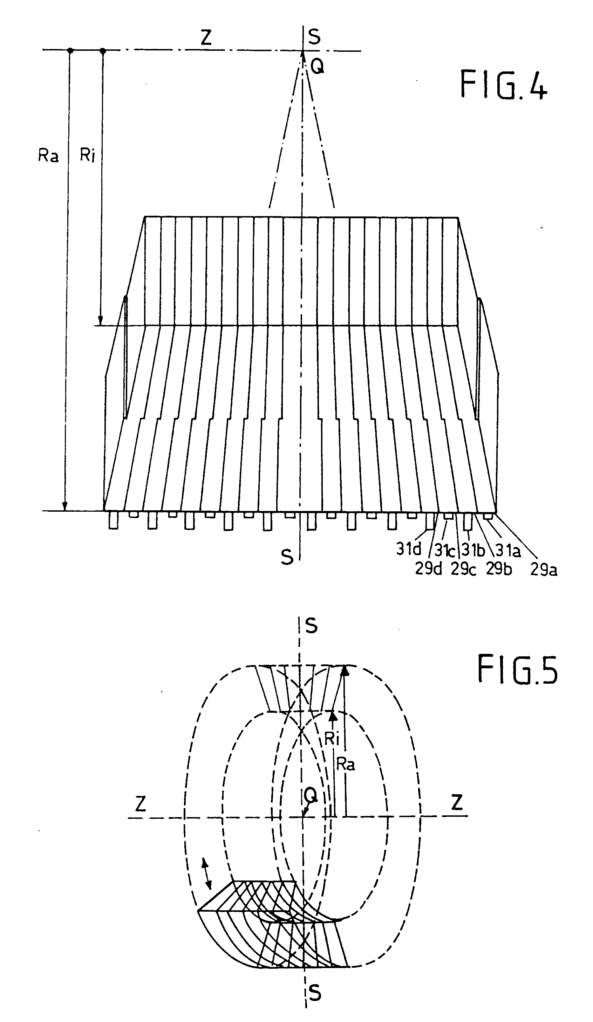

Fig. 4 zeigt eine Kollimatorhälfte mit 20 Blendplatten. Jede davon besitzt an ihrer Aussenfläche 29a, 29b,.. eine Zahnschiene 31a, 31b,.., in die das bereits erwähnte Schnecken-Zahnstangen-Getriebe eingreift. Die Zahnschienen 31a, 31b benachbarter Blendplatten haben jeweils eine unterschiedliche Höhe. Dadurch ist es möglich, die Schneckengetriebe auf dem wenigen, zur Verfügung stehenden Raum unterzubringen. Um für alle Blendplatten das gleiche Verhältnis von Motorendrehzahl zu Plattenvorschub zu erhalten, haben die Zahnschienen auch eine entsprechend verschiedene Steigung.

Die zwei Haltebügel einer Kollimatorhälfte sind an einer äusseren Bügelinnenseite kammförmig eingeschnitten. In die Einschnitte kommen die Zahnschienen zu liegen und auf benachbarten Zähnen des Kamms ruht jeweils die entsprechende Blendplatte mit ihrer Aussenfläche.

Die Blendplatten können natürlich auch mit anderen Mitteln gestützt und geführt werden. So können die Aussenflächen z.B. auch V-förmig ausgebildet und auf Rollen gelagert sein. Anstatt Zahnstangen an den Aussenflächen zu befestigen, können Stellmittel vorgesehen sein, welche direkt an der entsprechend geformten Aussenfläche angreifen.

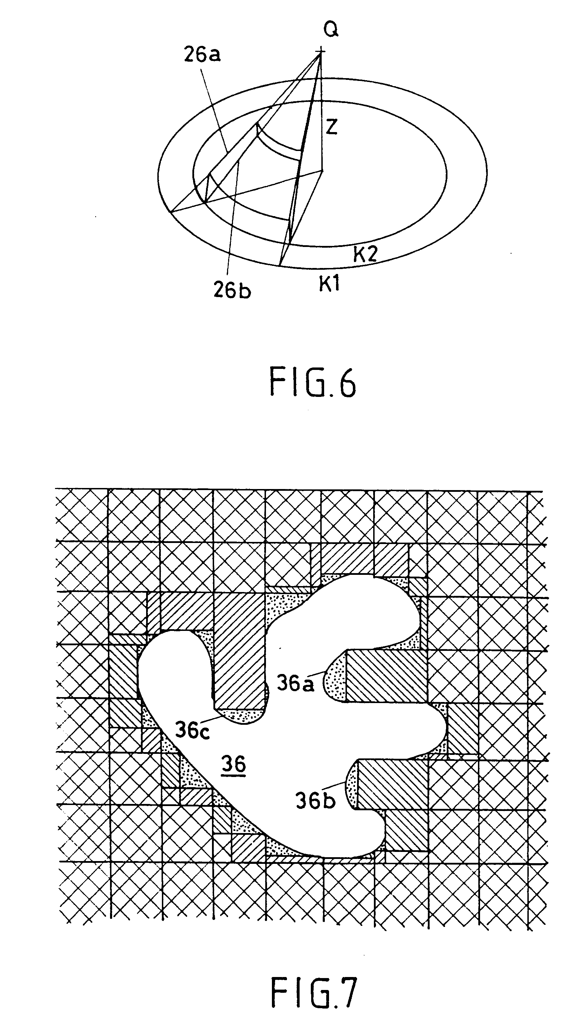

In Fig. 4 sind ausserdem Strahlachse S, Fokuspunkt Q, Kegelachse Z, sowie innerer Radius Ri und äusserer Radius Ra eingezeichnet. Die Bedeutung dieser geometrischen Bezugsgrössen wird anhand von Fig. 5 erklärt.

Fig. 5 zeigt eine räumliche Darstellung der Bewegungsbahnen der Blendplatte zur Erläuterung der Erfindung. Im Fokuspunkt Q schneiden sich die Strahlachse S und die Kegelachse Z unter einem rechten Winkel. Die Kegelachse Z ist Achse eines inneren und eines äusseren Zylinders mit Radius Ri resp. Ra, wobei Ri < Ra. Als Mittelebene wird eine Ebene senkrecht zur Kegelachse Z durch den Fokuspunkt Q definiert. Die Strahlachse S liegt also in der Mittelebene.

Der äussere Zylinder wird nun mit zur Mittelebene parallelen und zueinander äquidistanten Ebenen (Abstand b) geschnitten. Die so entstehenden Schnittkurven sind Kreise.

Für jeden Kreis wird eine Fläche definiert, die dadurch zustande kommt, dass die Spitze eines Vektors, welcher vom Fokuspunkt Q ausgeht, auf dem Kreis umläuft. Die so definierte Fläche ist jeweils ein Kegelmantel.

Jeder Kegelmantel schneidet auch den inneren Zylinder. Ein Raumgebiet, welches jeweils durch zwei benachbarte Kegelmäntel und durch den inneren und den äusseren Zylinder begrenzt ist, stellt die Bahn dar, auf welcher sich eine Blendplatte des erfindungsgemässen Konturenkollimator bewegt. Die Blendplatte selbst ist nichts anderes als ein winkelmässig begrenzter Ausschnitt (Segment) dieses Raumgebiets.

Aufgrund dieser geometrischen Ueberlegung wird folgendes klar:
1. Der erfindungsgemässe Konturenkollimator ist doppelt fokussiert. In jeder Stellung sind die Blendplatten sowohl mit den Seiten- als auch den Frontflächen nämlich so ausgerichtet, dass ein vom Fokuspunkt ausgehender Strahl sie nur tangieren, nicht aber schneiden kann.
2. Der Zusammenhang zwischen Plattenvorschub und Drehzahl des Motors ist linear. Die Aussenflächen der Blendplatten sind nämlich alle gleich lang und bewegen sich auf der gleichen Zylindermantelfläche.

Der äussere resp. innere Zylindermantel ist als Hüllfläche zu verstehen. D.h. die Aussen- und Innenflächen der Blendplatten brauchen nicht exakt Teil eines Zylindermantels zu sein. Es liegt durchaus im Bereich der Erfindung, diese Flächen zu Führungszwecken speziell auszuformen.

Fig. 6 zeigt eine dreidimensionale Darstellung einer Blendplatte, anhand welcher die gemäss dem Erfindungsgedanken notwendigen Merkmale der Erfindung herausgeschält werden sollen. Es sind deren zwei:
1. Jede Seitenfläche 26a,26b bildet einen Teil einer Mantelfläche eines Kegels K1,K2. Alle solchen Kegel K1,K2 haben ihre Spitze im Fokuspunkt Q. Ferner haben sie eine gemeinsame Kegelachse Z. Es folgt daraus, dass die beiden, zu einer Blendplatte gehörenden Kegel K1,K2 verschieden steil sind. Andererseits sind die Kegel von einander zugewandten Seitenflächen benachbarter Blendplatten identisch. Aus diesem Grund passen benachbarte Blendplatten formschlüssig aneinander.
2. Die Blendplatten müssen von geeigneten Mitteln so geführt sein, dass sie eine reine Rotation um die gemeinsame Kegelachse Z ausführen. Dadurch wird erreicht, dass sich die Blendplatten beim Verschieben nicht auseinanderbewegen. Die Seitenflächen nebeneinander angeordneter Blendplatten bleiben also auch in formschlüssigem Kontakt.

Diese beiden Kernpunkte der Erfindung bleiben von der sonstigen Form der Blendplatte (Aussenfläche, Innenfläche, gestufte Seitenfläche usw.) und der Art der Führungsmittel unberührt.

Fig. 7 zeigt eine Darstellung eines Strahlungsfeldes, wie es mit einem Strahlerkopf mit zwei Konturenkollimatoren erzeugt werden kann. Es wird eine Ebene senkrecht zur Strahlachse durch das Isozentrum dargestellt, sowie eine Projektion der Blendplatten auf diese Ebene. In der Mitte der Figur ist das zu bestrahlende Gebiet 36 eingezeichnet. Es hat z.B. mehrere Einbuchtungen 36a, 36b, 36c, welchen bei der Bestrahlung Rechnung getragen werden soll, und zwar in dem Sinne, dass sie möglichst vor der Strahlung geschützt sein sollen.

Die Blendplatten werden nun soweit vorgefahren, dass eine effektive Strahlungsfläche 33 dem zu bestrahlenden Gebiet 36 möglichst nahe kommt. In Fig. 7 ist mit der Schraffur angedeutet, dass
1. gewisse Gebiete (kariert) sowohl von oberen als auch vom unteren Konturenkollimator abgedeckt werden, und
2. gewisse Randgebiete (schräg schraffiert) nur von einem der beiden Konturenkollimatoren abgedeckt werden.

Bisher war es nötig, dass jeder einzelne Kollimator so hoch war, dass er das Strahlenbündel vollständig, d.h. bis auf ein gefordertes, unschädliches Niveau, abzuschwächen vermochte. Wenn aber wie in Fig. 2 zwei erfindungsgemässe Konturenkollimatoren eingesetzt werden, dann kann die Höhe d (siehe Fig. 3) der Blendplatten reduziert werden. Dies hat seinen Grund darin, dass jeder Punkt des Strahlungsfeldes sowohl vom oberen als auch vom unteren Konturenkollimator selektiv abgedeckt werden kann.

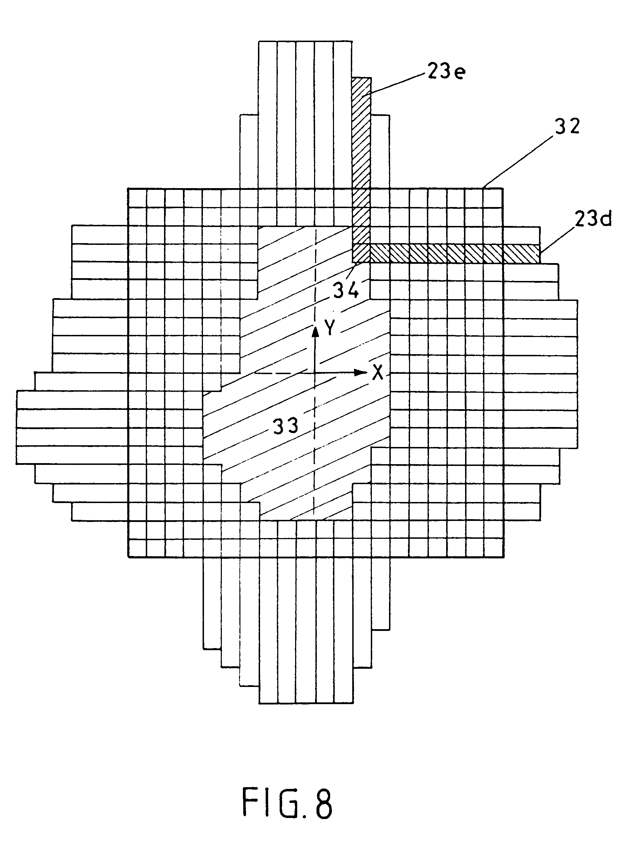

Fig. 8 veranschaulicht die Ansteuerung von Konturenkollimatoren mit reduzierter Höhe. Es wird dieselbe Ebene wie in Fig. 7 dargestellt.

Das Strahlungsfeld hat eine quadratische maximale Strahlungsfläche 32 von beispielsweise 40 cm x 40 cm. Unter der effektiven Strahlungsfläche 33 ist der Teil des Strahlungsfeldes zu verstehen, welcher nicht durch Blendplatten abgedeckt ist.

Im vorliegenden Beispiel ist jede der vier Kollimatorhälften aus 20 Blendplatten aufgebaut. Die Blendplatten des unteren Konturenkollimators bewegen sich in der Darstellung der Fig. 7 in X-Richtung und diejenigen des oberen Konturenkollimators in Y-Richtung (siehe eingezeichnetes Koordinatensystem). Die Dicke b der einzelnen Blendplatten ist so gewählt, dass ihre Zentralprojektion vom Fokuspunkt auf eine Ebene senkrecht zur Strahlachse S gleich gross ist. Die Blendplatten des oberen Konturenkollimators sind also etwas schmaler als diejenigen des unteren.

23d bezeichnet eine frei gewählte Blendplatte des unteren und 23e eine des oberen Konturenkollimators. Diese beiden Blendplatten kontrollieren miteinander einen Feldpunkt 34. Wenn also beide Blendplatten 23d und 23e wie in Fig. 7 gezeigt vorgeschoben sind, dann genügt es, wenn sie zusammen das Strahlungsfeld abzudecken vermögen. Gegenüber einem herkömmlichen Kollimator kann die Höhe d also verringert werden. Im Prinzip kann die Höhe auf die Hälfte reduziert werden.

Vorzugsweise ist die Höhe d gegenüber dem ursprünglichen Wert aber nur um etwa einen Drittel reduziert. Eine solche Höhe bewirkt nämlich bereits eine Reduktion der Intensität auf wenige Prozent. Bei einem zu bestrahlenden Gebiet wie z.B. in Fig. 7 können die zu schützenden Einbuchtungen 36a,36b,36c meist schon hinreichend gut vor Strahlenschäden bewahrt werden, obwohl sie nur durch die Blendplatten eines Konturenkollimators abgedeckt sind. (Die schräg schraffierten Gebiete, die ja mit konventionellen Kollimatorblöcken überhaupt nicht abgedeckt werden könnten, werden also nur mit wenigen Prozenten der Strahldosis belastet.)

Ein Strahlentherapiegerät mit Konturenkollimatoren mit reduzierter Höhe muss eine Steuerschaltung umfassen, die die Blendplatten so steuert, dass jeder abzudeckende Feldpunkt stets sowohl von einer Blendplatte des oberen als auch von einer des unteren Konturenkollimators abgedeckt ist. Eine solche Steuerschaltung kann am besten durch einen programmierten Mikroprozessor realisiert werden.

Der Vorteil dieser Ausführungsform liegt darin, dass im Strahlerkopf mehr Platz zur Verfügung steht oder umgekehrt die Bauhöhe kleiner wird und dass das Gewicht der Konturenkollimatoren kleiner wird.

Eine bevorzugte Ausführungsform der Erfindung umfasst eine Matrix-Ionisationskammer 19 zur Ueberwachung des Strahlungsfeldes.

Strahlentherapiegeräte sind gewissen Sicherheitsbestimmungen unterworfen. Dazu gehört z.B. auch die Vorschrift, dass die Kollimatoren über zwei getrennte Kanäle zu überwachen sind. Einer dieser Kanäle ist der Schrittmotor, welcher die Blendplatte in einer genau definierten Stellung festhält. Der andere Kanal ist nun die Matrix-Ionisationskammer 19 (siehe Fig. 1). Sie ist auf der Strahlachse S unterhalb des Isozentrums I angeordnet. Sie detektiert also die Form des Strahlungsfeldes wie es auf den Patienten einwirkt, und zwar nahezu in Echtzeit. Falls ein Fehler im Konturenkollimator auftritt, kann die Therapie sogleich unterbrochen werden.

Mit dieser Anordnung ist es auch möglich, das Strahlungsfeld während der Therapie zu verändern. Dies ist z.B. dann von Vorteil, wenn die Bestrahlungsrichtung während der Behandlung geändert wird (Rotation um Isozentrum I).

Die Matrix-Ionisationskammer ist als solche bekannt. Deshalb wird an dieser Stelle nicht weiter darauf eingegangen, sondern ausdrücklich auf die veröffentlichte europäische Patentanmeldung EP-A-0 196 138 verwiesen.

Die Erfindung lässt sich auf die verschiedensten Weisen verwirklichen. Im folgenden werden kurz einige weitere Möglichkeiten angedeutet.

Zur Ueberwachung der Blendplatten können natürlich auch Potentiometer eingesetzt werden. Diese können insbesondere alternativ die Ueberwachungsfunktion der Matrixionisationskammer wahrnehmen.

Die Reduktion der Leckstrahlung zwischen den Blendplatten kann statt mit den beschriebenen Stufen auch mit nutartigen Ausnehmungen erreicht werden. Es versteht sich dabei, dass die Ausnehmungen parallel zu Innen- und Aussenfläche verlaufen und einen im wesentlichen formschlüssigen Kontakt benachbarter Blendplatten erlauben.

Um die Zahl der zur Ansteuerung der Konturenkollimatoren benötigten Stellglieder möglichst klein zu halten, werden die Schrittmotoren mit Vorteil im Multiplex-Modus betrieben.

Die Blendplatten bestehen aus einem für Kollimatoren gängigen Material. Als Beispiel seien Wolframlegierungen genannt. Wenn nun zum Abdecken des Strahlungsfeldes eine Höhe d = 70 mm eines solchen Materials erforderlich sind, dann genügt bei der Ausführungsform mit reduzierter Höhe für einen Konturenkollimator eine Höhe von d = 35 mmm. Eine bevorzugte Höhe läge etwa bei 50 mm.

Abschliessend kann gesagt werden, dass die Erfindung ein vielseitig einsetzbares Strahlentherapiegerät schafft.

## Patentansprüche

1. Strahlentherapiegerät mit einem von einem Fokuspunkt her entlang einer Strahlachse sich ausbreitenden Strahlenbündel und einem auf der Strahlachse angeordneten Strahlerkopf mit folgenden Merkmalen:
a) der Strahlerkopf umfasst einen doppelt fokussierten Konturenkollimator (Multi Leaf Collimator);
b) der Konturenkollimator weist eine Mehrzahl von nebeneinander angeordneten Blendplatten auf, welche jeweils zwei Seitenflächen, zwei Frontflächen und eine Innen- und eine Aussenfläche haben;
c) es sind Mittel zum Verschieben jeder einzelnen Blendplatte vorhanden; dadurch gekennzeichnet, dass
d) jede Seitenfläche jeder Blendplatte einen Teil einer Mantelfläche eines Kegels bildet, wobei alle solchen Kegel sowohl eine gemeinsame Kegelachse, welche senkrecht zur Strahlachse durch den Fokuspunkt verläuft, als auch eine gemeinsame Kegelspitze, welche mit dem Fokuspunkt zusammenfällt, haben, und
e) dass Mittel zum Führen der Blendplatten vorgesehen sind, sodass jede Blendplatte beim Verschieben eine reine Rotation um die Kegelachse ausführt.

2. Strahlentherapiegerät nach Anspruch 1, dadurch gekennzeichnet, dass
a) die Aussenflächen aller seitlich nebeneinander angeordneten Blendplatten insgesamt als Hüllfläche einen Teil einer Mantelfläche eines äusseren Zylinders haben, dessen Achse die Kegelachse ist, und dass
b) die Mittel zum Verschieben der Blendplatten an den Aussenflächen angreifen.

3. Strahlentherapiegerät nach Anspruch 1, dadurch gekennzeichnet, dass
a) die Innenflächen aller seitlich nebeneinander angeordneten Blendplatten insgesamt als Hüllfläche einen Teil einer Mantelfläche eines inneren Zylinders haben, dessen Achse die Kegelachse ist, und dass
b) die Mittel zum Führen von nebeneinander angeordneten Blendplatten (23) mindestens zwei Haltebügeln (24b,24c) umfassen, sodass jede Blendplatte an mindestens vier Stellen gestützt ist.

4. Strahlentherapiegerät nach Anspruch 2, dadurch gekennzeichnet, dass die Mittel zum Verschieben für jede Blendplatte (23)
a) eine an der Aussenfläche der Blendplatte (23) angebrachte Verstellhilfe,
b) ein darin eingreifendes Getriebe und
c) einen dieses Getriebe betätigenden Antrieb umfassen.

5. Strahlentherapiegerät nach Anspruch 4, dadurch gekennzeichnet, dass
a) die Verstellhilfe eine an der Aussenfläche befestigte Zahnschiene,
b) das Getriebe ein selbsthemmender Schnecken-Zahnstangen-Antrieb und
c) der Antrieb ein Schrittmotor ist.

6. Strahlentherapiegerät nach Anspruch 1, dadurch gekennzeichnet, dass der Strahlerkopf (3) zwei übereinander angeordnete und senkrecht zueindander ausgerichtete Konturenkollimatoren aufweist.

7. Strahlentherapiegerät nach Anspruch 6, dadurch gekennzeichnet, dass die Konturenkollimatoren eine reduzierte Höhe haben, wobei die reduzierte Höhe eines einzelnen Konturenkollimators nicht genügt, die Summe der Höhen der übereinander angeordneten Konturenkollimatoren aber genügt, um das Strahlenbündel auf ein gefordertes Mass abzuschwächen.

8. Strahlentherapiegerät nach Anspruch 1, dadurch gekennzeichnet, dass auf der Strahlachse (S) gegenüber dem Strahlerkopf (3) und hinter einem Isozentrum eine Matrix-Ionisationskammer angeordnet ist, mit welcher die Konturenkollimatoren überwacht werden.

9. Strahlentherapiegerät nach Anspruch 5, dadurch gekennzeichnet, dass die Zahnschienen benachbarter Blendplatten eines Konturenkollimators eine unterschiedliche Höhe und eine dadurch bedingte unterschiedliche Steigung aufweisen, wobei die Steigung derart ausgelegt ist, dass zwischen Motorendrehzahl und Plattenvorschub stets ein gegebener, für alle Blendplatten gültiger, linearer Zusammenhang besteht.

10. Strahlentherapiegerät nach Anspruch 6, dadurch gekennzeichnet, dass die
a) Konturenkollimatoren eine reduzierte Höhe haben, wobei die reduzierte Höhe eines einzelnen Konturenkollimators nicht genügt, die Summe der Höhen der übereinander angeordneten Konturenkollimator aber genügt, um das Strahlenbündel auf ein gefordertes Mass abzuschwächen, und dass
b) eine Steuerschaltung vorgesehen ist, welche die einzelnen Blendplatten der Konturenkollimatoren so positioniert, dass ein abzuschirmender Feldpunkt (34) des Strahlenbündels stets von zwei Blendplatten (23d,23e) abgedeckt ist.

11. Strahlentherapiegerät nach Anspruch 6 oder 10, dadurch gekennzeichnet, dass die reduzierte Höhe etwa zwei Drittel der zur geforderten Abschwächung des Strahlenbündels nötigen Höhe beträgt.

## Claims

1. Radiation therapy unit with a beam of rays propagating from a focal point along a beam axis and a radiator head arranged on the beam axis, having the following features:
a) the radiator head comprises a double-focus multi-leaf collimator;
b) the multi-leaf collimator exhibits a plurality of adjacently arranged diaphragm plates which in each case have two side faces, two front faces and an inside and an outside face;
c) means are provided for displacing each individual diaphragm plate; characterized in that
d) each side face of each diaphragm plate forms a part of a surface area of a cone, all such cones having both a common cone axis which extends perpendicularly to the beam axis through the focal point, and a common cone point which coincides with the focal point, and
e) means are provided for guiding the diaphragm plates so that each diaphragm plate performs a pure rotation about the cone axis during its displacement.

2. Radiation therapy unit according to Claim 1, characterized in that
a) the outside faces of all diaphragm plates arranged laterally adjacently have overall as an enveloping surface a part of a surface area of an outer cylinder the axis of which is the cone axis, and in that
b) the means for displacing the diaphragm plates engage the outside faces.

3. Radiation therapy unit according to Claim 1, characterized in that
a) the inside faces of all diaphragm plates arranged laterally adjacently have overall as an enveloping surface a part of a surface area of an inner cylinder the axis of which is the cone axis, and in that
b) the means for guiding adjacently arranged diaphragm plates (23) comprise at least two holding yokes 24b, 24c) so that each diaphragm plate is supported at at least four points.

4. Radiation therapy unit according to Claim 2, characterized in that the means for displacing comprise for each diaphragm plate (23)
a) an adjusting aid attached to the outside face of the diaphragm plate (23),
b) a gear which engages this adjusting aid and
c) a drive actuating this gear.

5. Radiation therapy unit according to Claim 4, characterized in that
a) the adjusting aid is a toothed rail mounted on the outside face,
b) the gear is a self-inhibiting worm-rack drive and
c) the drive is a stepping motor.

6. Radiation therapy unit according to Claim 1, characterized in that the radiator head (3) exhibits two multi-leaf collimators which are arranged one above the other and are aligned perpendicularly with respect to one another.

7. Radiation therapy unit according to Claim 6, characterized in that the multi-leaf collimators have a reduced height, the reduced height of a single multi-leaf collimator not being sufficient but the sum of the heights of the multi-leaf collimators arranged above one another being sufficient for attenuating the beam of rays to a required level.

8. Radiation therapy unit according to Claim 1, characterized in that a matrix ionization chamber, by means of which the multi-leaf collimators are monitored, is arranged on the beam axis (S) opposite the radiator head (3) and behind an isocentre.

9. Radiation therapy unit according to Claim 5, characterized in that the toothed rails of adjacent diaphragm plates of a multi-leaf collimator exhibit a different height and a resultant different pitch, the pitch being designed in such a manner that a given linear relationship which is valid for all diaphragm plates always exists between motor speed and plate advance.

10. Radiation therapy unit according to Claim 6, characterized in that the
a) multi-leaf collimators have a reduced height, the reduced height of a single multi-leaf collimator not being sufficient but the sum of the heights of the multi-leaf collimators arranged one above the other being sufficient for attenuating the beam of rays to a required level and in that
b) a control circuit is provided which positions the individual diaphragm plates of the multi-leaf collimators in such a manner that a field point (34) to be shielded in the beam of rays is always covered by two diaphragm plates (23d, 23e).

11. Radiation therapy unit according to Claim 6 or 10, characterized in that the reduced height is about two thirds of the height needed for the required attenuation of the beam of rays.

## Revendications

1. Appareil de radiothérapie avec un faisceau de rayonnement se propageant le long d'un axe de rayonnement depuis un foyer ponctuel et avec une tête émettrice de rayonnement disposée sur l'axe de rayonnement, ayant les caractéristiques suivantes:
a) la tête émettrice de rayonnement comprend un diaphragme multiplans à double foyer (Multi-Leaf Collimator);
b) le diaphragme multiplans présente une pluralité de plaques collimatrices disposées les unes à côté des autres, lesquelles ont chacune deux faces latérales, deux faces frontales, une face intérieure et une face extérieure;
c) il est prévu des moyens pour déplacer chaque plaque collimatrice séparément; caractérisé en ce que
d) chaque face latérale de chaque plaque collimatrice constitue une partie de la surface latérale d'un cône, tous ces cônes ayant non seulement un axe de cône commun, qui s'étend perpendiculairement à l'axe de rayonnement et passe par le foyer ponctuel, mais aussi un sommet de cône commun, qui coïncide avec le foyer ponctuel, et
e) il est prévu des moyens de guidage des plaques collimatrices, de sorte que chaque plaque collimatrice effectue, lors du déplacement, une simple rotation autour de l'axe du cône.

2. Appareil de radiothérapie selon la revendication 1, caractérisé en ce que
a) les faces extérieures de toutes les plaques collimatrices disposées latéralement les unes à côté des autres ont globalement en tant que surface enveloppante, la forme d'une portion de la surface latérale d'un cylindre extérieur, dont l'axe est l'axe du cône, et
b) les moyens pour le déplacement des plaques collimatrices s'engagent avec les faces extérieures.

3. Appareil de radiothérapie selon la revendication 1, caractérisé en ce que
a) les faces intérieures de toutes les plaques collimatrices disposées latéralement les unes à côté des autres ont globalement en tant que surface enveloppante, la forme d'une portion de la surface latérale d'un cylindre intérieur, dont l'axe est l'axe du cône, et
b) les moyens de guidage des plaques collimatrices (23) disposées les unes à côté des autres comprennent au moins deux étriers de support (24b, 24c), de sorte que chaque plaque collimatrice soit supportée en au moins quatres endroits.

4. Appareil de radiothérapie selon la revendication 2, caractérisé en ce que les moyens pour déplacer chaque plaque collimatrice (23) comprennent
a) un élément auxiliaire de déplacement placé à la face extérieure de la plaque collimatrice (23),
b) un engrenage s'engrènant dans celui-ci et
c) un entraînement actionnant cet engrenage.

5. Appareil de radiothérapie selon la revendication 4, caractérisé en ce que
a) l'élément auxiliaire de déplacement est une crémaillère fixée à la face extérieure,
b) l'engrenage est un entraînement à crémaillère à vis sans fin autobloquant et
c) l'entraînement est un moteur pas à pas.

6. Appareil de radiothérapie selon la revendication 1, caractérisé en ce que la tête émettrice de rayonnement (3) présente deux diaphragmes multiplans disposés l'un au-dessus de l'autre et perpendiculairement l'un à l'autre.

7. Appareil de radiothérapie selon la revendication 6, caractérisé en ce que les diaphragmes multiplans ont une hauteur réduite, la hauteur réduite d'un diaphragme multiplans unique n'étant pas suffisante pour affaiblir le faisceau de rayonnement dans la mesure souhaitée, mais la somme des hauteurs des diaphragmes multiplans disposés l'un sur l'autre étant suffisante.

8. Appareil de radiothérapie selon la revendication 1, caractérisé en ce qu'une chambre d'ionisation matricielle est disposée sur l'axe de rayonnement (S) en face de la tête émettrice de rayonnement (3) et derrière un isocentre, cette chambre permettant de surveiller les diaphragmes multiplans.

9. Appareil de radiothérapie selon la revendication 5, caractérisé en ce que les crémaillères de plaques collimatrices adjacentes d'un diaphragme multiplans ont une hauteur différente et par conséquent une augmentation différente, l'augmentation étant déterminée de telle sorte qu'il existe toujours une relation linéaire donnée, valide pour toutes les plaques collimatrices, entre la vitesse des moteurs et l'avance des plaques.

10. Appareil de radiothérapie selon la revendication 6, caractérisé en ce que
a) les diaphragmes multiplans ont une hauteur réduite, la hauteur réduite d'un diaphragme multiplans unique n'étant pas suffisante pour affaiblir le faisceau de rayonnement dans la mesure souhaitée, mais la somme des hauteurs des diaphragmes multiplans disposés l'un sur l'autre étant suffisante, et
b) il est prévu un circuit de commande qui positionne les plaques collimatrices individuelles des diaphragmes multiplans de telle sorte, qu'un point du champ (34) à protéger dans le faisceau de rayonnement est toujours recouvert de deux plaques collimatrices (23d, 23e).

11. Appareil de radiothérapie selon la revendication 6 ou 10, caractérisé en ce que la hauteur réduite vaut environ deux tiers de la hauteur nécessaire à l'affaiblissement souhaité du faisceau de rayonnement.
